# EUROPEAN PATENT APPLICATION

(11) **EP 1 092 446 A2**
(43) Date of publication of application: **18.04.2001**
(21) Application number: 00308962.0
(22) Date of filing: 12.10.2000
(51) Int. Cl.: A61M 15/00

(54) **Nebuliser**

(30) Priority: 12.10.1999 GB 9924158
(71) Applicant: Lifecare Designs Limited, Birmingham, West Midlands B23 6NP (GB)
(72) Inventor: Hopkins, Andrew David, Sutton Coldfield, West Midlands B73 5EE (GB)
(74) Representative: Goodenough, Nigel

(57) **Abstract**

The present invention relates to a nebuliser comprising an electrically energisable ultrasonic transducer having an oscillation frequency dependent current and voltage characteristic exhibiting the occurrence of a minimum magnitude of current through the transducer at an anti-resonance frequency of the transducer, the voltage across the transducer increasing with an increase in frequency within a range of frequencies including said anti-resonance frequency. The nebuliser further comprises a transducer drive system for generating a drive signal for energising the transducer, the drive system comprising oscillation frequency control means operable to maintain the frequency of the drive signal at a specific frequency within said range of frequencies by reference to the variation of both current through the transducer and voltage across the transducer with a change of frequency within the said frequency range. The transducer is arranged to receive said drive signal and to cause physical vibrations in fluid to be nebulised when energised by said drive signal. A transducer having a simple automatic frequency tuning system is thereby provided.

## Description

The present invention relates to a nebuliser and, particularly, but not exclusively, to an ultrasonic nebuliser suitable for nebulising/atomizing liquid based medication for the purpose of inhalation by a patient.

It is well known for liquid nebulisers to employ ultrasonic technology to provide a required degree of liquid atomization. Conventional ultrasonic nebulisers incorporate a piezo-oscillator element (i.e. a piezo-electric crystal) which is exposed to a liquid to be atomized and selectively excited by means of an electric oscillating circuit. Upon excitation, the piezo-oscillator element acts as a transducer and generates ultrasonic waves in said liquid which are directed to the liquid surface. As a consequence, the surface of said liquid is atomized into a fine mist which may be transported, as necessary, by suitable means such as an electric fan or simply by the inhalation of a patient.

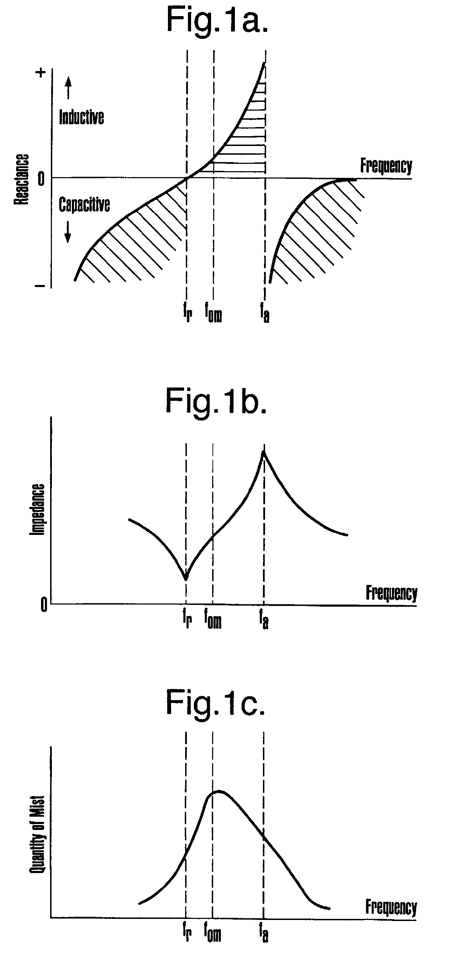

The characteristics of piezo-oscillator elements are well known and must be carefully considered when designing the oscillating circuit of a nebuliser if efficient liquid atomization is to be achieved. Both the electrical and mist generating characteristics of a typical piezo-oscillator element are schematically shown in Figures 1a, 1 b and 1 c of the accompanying drawings. It will be seen by reference to Figure 1 a that the effective reactance of a piezo-oscillator element is inductive (i.e. positive) at frequencies between a series (resonance) frequency fr and a parallel (anti-resonance) frequency fₐ of the transducer. At other frequencies, the effective reactance of the piezo-oscillator element is capacitive (i.e. negative). Also, it will be seen from Figure 1b that the effective impedance of a piezo-oscillator element is minimised at the resonance frequency fᵣ and maximised at the anti-resonance frequency fₐ.

The oscillation amplitude of a piezo-oscillator element is maximised at the resonance frequency fᵣ, however it will be seen from Figure 1c that this frequency fᵣ is somewhat less than the optimum frequency fₒₘ for maximising liquid atomization. Indeed, the optimum frequency fₒₘ for maximum yield of mist is between the resonance frequency fᵣ and the anti-resonance frequency fₐ.

Notwithstanding the fact that the optimum frequency in terms of mist generation is between the resonance and anti-resonance frequencies, the majority of conventional ultrasonic nebulisers incorporate oscillating circuitry which uses one of these frequencies as a basis for exciting the piezo-oscillating element. Typically, the oscillating circuitry is designed to automatically tune to one of said frequencies so that variations in, for example, ambient conditions, may be conveniently accommodated. A number of methods are employed for achieving this automatic tuning, one of which uses the voltage and current phases in respect of a piezo-oscillating element in conjunction with a phase comparator (see, for example, US 3,819,961). However, a problem associated with the prior art systems for controlling the oscillation frequency of piezo-oscillating elements is that such systems are generally relatively complicated.

In order for a medication dispensed by a nebuliser to be adequately received by a patient, it is important for the atomised liquid particles to have an appropriate size. Release of undesirably large particles is prevented in many prior art nebulisers by means of a baffle system. However, the effectiveness of such systems is heavily dependent upon fluid flow velocity and the resultant impinging of particles on one or more baffles tends to leave a large proportion of medication which is not inhaled. This reduces the overall effectiveness and efficiency of prior art nebulisers.

It is an object of the present invention to provide an ultrasonic nebuliser comprising means for automatically tuning to a required oscillation frequency wherein said means is comparatively simple and may be readily and inexpensively manufactured.

It is also an object of the present invention to provide a nebuliser incorporating effective and efficient means for controlling the size of atomised particles.

A first aspect of the present invention provides a nebuliser comprising an electrically energizable ultrasonic transducer having an oscillation frequency dependent current and voltage characteristic exhibiting the occurrence of a minimum magnitude of current through the transducer at an anti-resonance frequency of the transducer, the voltage across the transducer increasing with an increase in frequency within a range of frequencies including said anti-resonance frequency; a transducer drive system for generating a drive signal for energizing the transducer, the drive system comprising oscillation frequency control means operable to maintain the frequency of the drive signal at a specific frequency within said range of frequencies by reference to the variation of both current through the transducer and voltage across the transducer with a change of frequency within the said frequency range, the transducer being arranged to receive said drive signal and to cause physical vibrations in the fluid to be nebulised when energized by said drive signal.

It is preferable for the oscillation frequency control means to maintain the frequency of the drive signal at a magnitude at which the variation with frequency of a function of current through the transducer is zero. It is also preferable for the function of current to have a zero variation with frequency at a frequency magnitude other than the anti-resonance frequency of the transducer. The function of current may have a zero variation with frequency at a frequency magnitude at which the yield of fluid nebulised by the transducer is substantially maximised.

The oscillation frequency control means may comprise a comparator for determining the direction of change with frequency of the function of current through the transducer. Furthermore, the oscillation frequency control means may also comprise a comparator for determining the direction of change with frequency of voltage across the transducer. Preferably, outputs from the two comparators are applied to an exclusive OR gate for determination of the frequency magnitude at which the function of current has a zero variation with frequency. The function of current may be such that the relationship of said function with frequency is identical to that of current through the transducer with frequency.

A second aspect of the present invention provides a nebuliser comprising means for inducing a swirling motion in nebulised fluid about a swirl axis as said nebulised fluid is extracted from the nebuliser, the angular velocity of the swirling fluid being sufficient to result in undesirably large nebulised particles moving in a direction offset to the direction of swirl and thereby away from the swirl axis. Thus, oversized nebulised/atomised particles are effectively thrown from the swirling fluid. These oversized particles may be then collected and returned to a fluid reservoir.

The means for inducing a swirling motion in nebulised fluid preferably comprises one or more guides for deflecting a flow of fluid. The or each guide may be a curved guide vane.

The nebuliser preferably comprises a first chamber, in which fluid is nebulised, and a second chamber having an inlet and outlet and in which the means for inducing a swirling motion is arranged so as to induce a swirling motion in fluid passing through the second chamber from said inlet to said outlet; the first and second chambers being in fluid communication so that, when in use, swirling fluid in the second chamber tends to draw nebulised fluid into said second chamber from the first chamber. The means by which the first and second chambers are in fluid communication preferably comprises a passage joining the first and second chambers wherein said passage is centred on the swirl axis. The passage may comprise an aperture in a wall common to both the first and second chambers. Means may be provided for allowing fluid to flow into the first chamber as nebulised fluid is drawn into the second chamber.

The nebuliser preferably comprises means for collecting undesirably large nebulised particles which have moved away from the swirl axis. Said collection means preferably directs collected particles to the first chamber for re-nebulisation.

The first chamber preferably comprises a tube located above the surface of liquid to be nebulised so that the interior of the tube receives the majority of any nebulised fluid. One or more slots may be provided in the wall of the tube so as to allow nebulised fluid to pass from the interior of the tube to the exterior thereof.

A third aspect of the present invention provides a nebuliser comprises a demand prediction system which determines the period of a cyclical demand for nebulised fluid by monitoring fluid flow with detection means so as to determine the start of consecutive demand cycles and compensating for lag between the actual start of a demand cycle and the detected start of said cycle by calculating the start of a next demand cycle as being a length of time from the detected start of a current demand cycle where said length of time is equal to the determined period less a fixed length of time.

Said fixed length of time may be equal to or greater than the length of time between the actual start of a demand cycle and the detected start of said cycle. The detection means preferably comprises a thermistor.

An embodiment of the present invention will now be described with reference to the accompanying drawings, in which:
Figures 1a, 1b and 1c respectively show the variation with frequency of reactance, impedance and quality of mist generation of a piezo-oscillator element;
Figure 2 shows a schematic perspective view of a nebuliser according to the present invention;
Figure 3 shows a schematic partial perspective view of the nebuliser shown in Figure 2;
Figure 4 shows graphically the basis for operation of a breath prediction system;
Figure 5 shows the variation with frequency of voltage across a transducer (i.e. a piezo-oscillator element) and of current through a transducer;
Figure 6 is a block diagram showing the structure of the electric circuit of the nebuliser shown in Figure 2;
Figure 7 is a diagram of the control circuitry of the nebuliser shown in Figure 2;
Figure 8 is a diagram of the power supply circuitry of the nebuliser shown in Figure 2; and
Figure 9 is a block diagram showing the interface between the control circuitry of Figure 7 and the power supply circuitry of Figure 8.

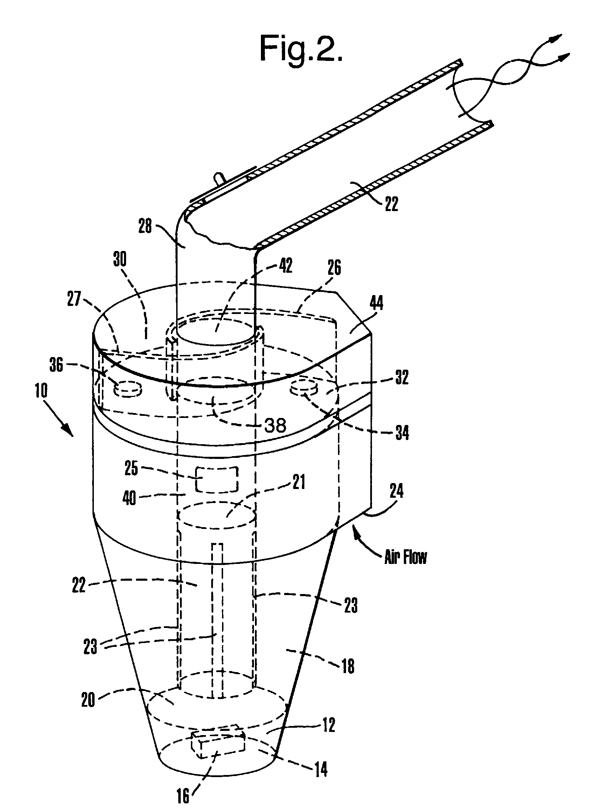

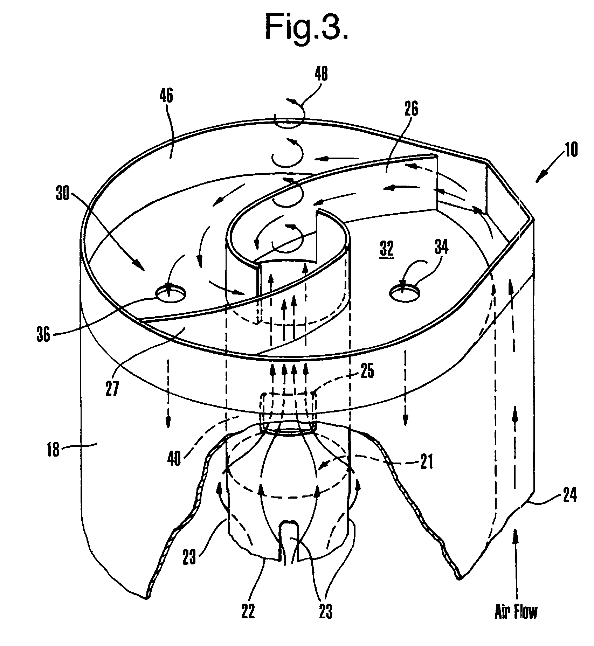

A nebuliser 10 according to the present invention incorporates a reservoir 12 for retaining a liquid based medication 14 to be nebulised (see Figures 2 and 3). This liquid 14 may consist of a solution or suspension of solid compounds. As is known in the art, an ultrasonic transducer 16 (i.e. a piezo-oscillator element) is mounted relative to the reservoir 12 so as to be exposed to the liquid medication 14. The transducer 16 is itself connected to appropriate electrical control circuitry (not shown in Figures 2 and 3) for providing excitation energy at a required frequency. The control circuitry is powered by means of rechargeable batteries.

A nebulising chamber 18 is located above the surface 20 of the liquid medication 14 so as to receive liquid which has been atomized. A mouthpiece 22 extends into the nebulising chamber 18 so as to allow a user of the nebuliser 10 (i.e. a patient) to inhale any atomized medication produced following excitation of the transducer 16. The lower end of the mouthpiece 22 is located adjacent the transducer 16 so as to receive all (or a substantial proportion) of the atomized medication generated during excitation. Atomized mediation is generally ejected from the surface of the liquid 14 as a fountain of small particles. In order to prevent these particles from being undesirably ejected from the nebuliser 10 through the mouthpiece 22, a barrier member 21 is located in the mouthpiece 22. The barrier member 21 is in the form of a concave dome and completely blocks the passage of fluid through the mouthpiece 22. In use, atomized medication to be inhaled by a patient bypasses the barrier member 21 by flowing from a location in the mouthpiece 22 below said member 21 into the nebulising chamber 18 via one of four elongate slots 23. The slots 23 are equi-spaced about the circumference of the mouthpiece 22. The atomized medication then flows through one of two apertures 25 to a location in the mouthpiece 22 above the barrier member 21. The two apertures 25 are located on opposite sides of the mouthpiece 22.

An inlet port 24 (provided as necessary with an appropriate one way valve) ensures that fluid pressure within the nebuliser 10 is equalised with the atmospheric pressure external to the nebuliser 10 as atomized medication is inhaled.

The nebuliser 10 further incorporates breath actuation means (not shown in Figures 2 and 3) in the form of electric circuitry which ensures that atomized medication is generated only as and when it is required by the user. In this way, the components of the nebuliser 10 are not used unnecessarily and, accordingly, the efficiency of the atomization process is improved and the service life of the nebuliser 10 is extended. The breath actuation means of the present embodiment incorporates a heated thermistor exposed to the flow path of atomized liquid drawn from the nebulising chamber 18. As a fluid flow is initiated upon the inhalation of a patient, the heated thermistor is cooled. This produces a change in thermistor resistance. Excitation of the transducer 16 may be thereby triggered.

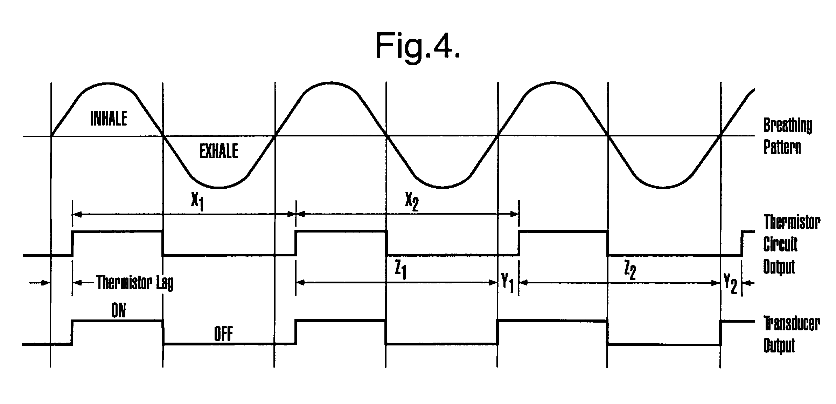

Due to the thermal lag of a thermistor, there is a small delay between the start of inhalation and the detection of a breath. Also, once a transducer is excited, there is a small delay before atomised medication is generated. Accordingly, it is preferable for the breath actuation means of the nebuliser 10 to be turned on for a fixed pre-determined period before actual detection of a breath. With reference to Figure 4, it will be seen that this is achieved by measuring the period X of a user's previous breathing cycle and using this measured time, less the fixed pre-determined delay time Y, to turn on the nebuliser after a calculated period Z (wherein Z = X - Y) from the detected start of the present breath. This is repeated continually for each breath and, for a series of breaths having the same period, results in the nebuliser being turned on at the same as the user begins to inhale. It will however be appreciated that the first two breaths of the user allow a calculation of the breath period X and, accordingly, anticipation of the user's inhalations is only made for the user's third and subsequent breaths. The continual monitoring of each breath allows adjustments to be made for variations in the user's rate of breathing. In other words, the system allows for variations in the breath period X. Also, by increasing the pre-determined delay time Y, the system may be configured to initiate excitation of the transducer slightly before the anticipated inhalation of a user so as to pre-fill the nebulising chamber 18 with atomised medication. This results in a faster treatment time. Furthermore, because of the characteristics of a thermistor as a flow sensor, nebulisation may be terminated before the end of inhalation so as to reduce the amount of medication being re-breathed into the atmosphere. This demand prediction system is preferably implemented by means of appropriate electronic circuitry.

The breath actuation means may be designed so that, once initiated, atomization continues until the heated thermistor senses a temperature change indicating the end of patient inhalation. Alternatively, the breath actuation means may be designed so that atomization is terminated when the heated thermistor senses a decrease in breathing rate to, for example, 80% of the maximum breathing rate detected during the current breath. In yet further designs of breath actuation means, atomization may be terminated after a fixed pre-determined time period or after a period dependent upon the length of the previous breath. Termination of atomization may also be made dependent upon the period of time taken for the thermistor to detect a pre-determined change in the peak value measured during the current breath or the average value of the previous breaths, wherein said pre-determined change is a pre-determined percentage of said peak value. Breath actuation means employing a heated thermistor to detect fluid flow is disclosed in EP 0 178 925 A2.

The present embodiment also incorporates means for detecting when the reservoir 12 has been emptied (or is about to be emptied) of the liquid based medication 14 and for switching off the nebuliser 10 upon such detection and indicating to the user that the source of medication has been exhausted (or is about to be exhausted). The means for detecting exhaustion of the medication source incorporates control logic which compares the current passing through the transducer with pre-determined values of current magnitude corresponding to a minimum acceptable level of liquid medication. As the reservoir 12 is emptied, the transducer 16 becomes unloaded and the current passing therethrough reduces. Accordingly, the control logic can be conveniently designed to switch off the nebuliser once the magnitude of the transducer current has dropped to the pre-determined level. At this stage, an LED (i.e. a Light Emitting Diode) may be activated by said control logic so as to provide the required indication to the user. Appropriate means for detecting a minimum acceptable level of liquid within the reservoir 12 is disclosed in GB 2,219,512 A.

In order for the medication dispensed by the nebuliser 10 to be adequately received by a patient, it is important that the particles of the atomized liquid have an appropriate size. Ideally, the diameter of the particles should be between 0.5 :m and 5.0 :m. Accordingly, as in many prior art nebuliser designs, the present embodiment is provided with a transducer capable of generating maximum quantities of mist in a frequency range of approximately 1 to 2.5 MHz. This frequency range produces particles of the desired size. In addition, many prior art nebulisers limit particle size by means of a baffle system. However, the effectiveness of such systems is heavily dependent upon fluid flow velocity and the resultant impinging of particles on one or more baffles tends to result in a large proportion of medication (i.e. residual medication) not being inhaled by the patient.

In order to overcome this problem, the present nebuliser 10 incorporates two vanes 26,27 (although a single vane may be sufficient) for generating fluid swirl in an upper portion 28 of the mouthpiece 22. The two vanes 26,27 are located in an upper chamber 30 situated above the nebulising chamber 18. A wall 32 common to both the upper chamber 30 and the nebulising chamber 18 is provided with two apertures 34,36 which, in use, permit air to flow into the nebulising chamber 18 from the upper chamber 30. A central aperture 38 is also provided in the wall 32 for permitting a flow of air and atomised medication to pass from the nebulising chamber 18 into the upper chamber 30 via a lower portion 40 of the mouthpiece 22. A second central aperture 42 is defined opposite said first central aperture 38 in an upper wall 44 (not shown in Figure 3) of the nebuliser 10. The upper portion 28 of the mouthpiece 22 extends from the second central aperture 42 outwardly from the upper chamber 30. The mouthpiece 22 does not extend through the upper chamber 30. Furthermore, the two vanes 26,27 are arranged to generate a swirling flow of fluid in the region between the two central apertures 38,42.

The arrangement is such that, in use, inhalation by a patient through the upper portion 28 of the mouthpiece 22 causes air to enter the nebuliser 10 at the inlet port 24. By reference to Figure 3, it will be seen that the intake of air progresses into the upper chamber 30 where it is separated into two distinct flows by a first vane 26. The vanes 26,27 and a curved side wall 46 of the upper chamber 30 are such that the separated air flows are directed to the centre of the upper chamber 30 between the two central apertures 38,42. The air flows are so directed that a swirling motion is induced therein. Vortices 48 are formed in the region of the central apertures 38,42 which, due to their lower static fluid pressure, tend to draw a mixture of air and atomised medication from the nebulising chamber 18 into the upper chamber 30 via the lower portion 40 of the mouthpiece 22. This fluid flow from the nebulising chamber 18 results in a flow of air into the nebulising chamber 18 from the upper chamber 30 via the apertures 34,36. The vortices 48 and atomised medication drawn into the upper chamber 30 are inhaled by the patient via the upper portion 28 of the mouthpiece 22.

The spinning fluid flow rotates with sufficient velocity for undesirably large particles of atomised medication to be effectively thrown against the vanes 26,27 (and/or to the sides of the mouthpiece 22). Said large particles of medication are then received in a skirt or gutter (not shown) and thereby directed back to the reservoir 12 under the action of gravity. In this way, the amount of residual medication arising through use of the nebuliser 10 is minimised.

With regard to the control of residual medication, the mouthpiece of an alternative embodiment may be provided as two concentrically mounted tubes wherein atomized medication is inhaled by a patient through the inner tube whilst pressure equalisation within the nebuliser is achieved by virtue of an air flow into the nebuliser which passes through a generally annular shaped passage formed between said tubes. Thus, large particles leaving the nebuliser chamber tends to become entrained in the incoming air flow. This further obviates the need for an internal baffle system.

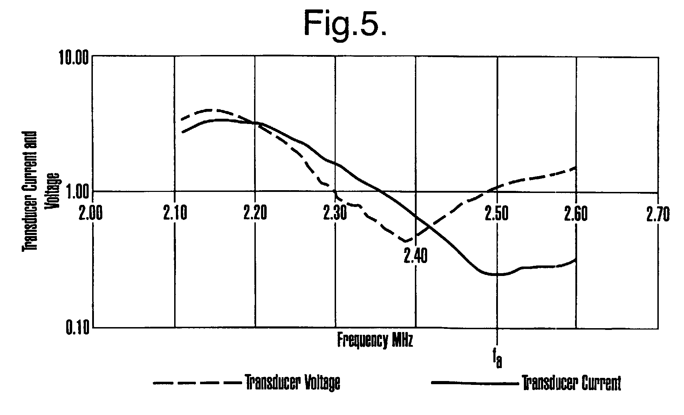

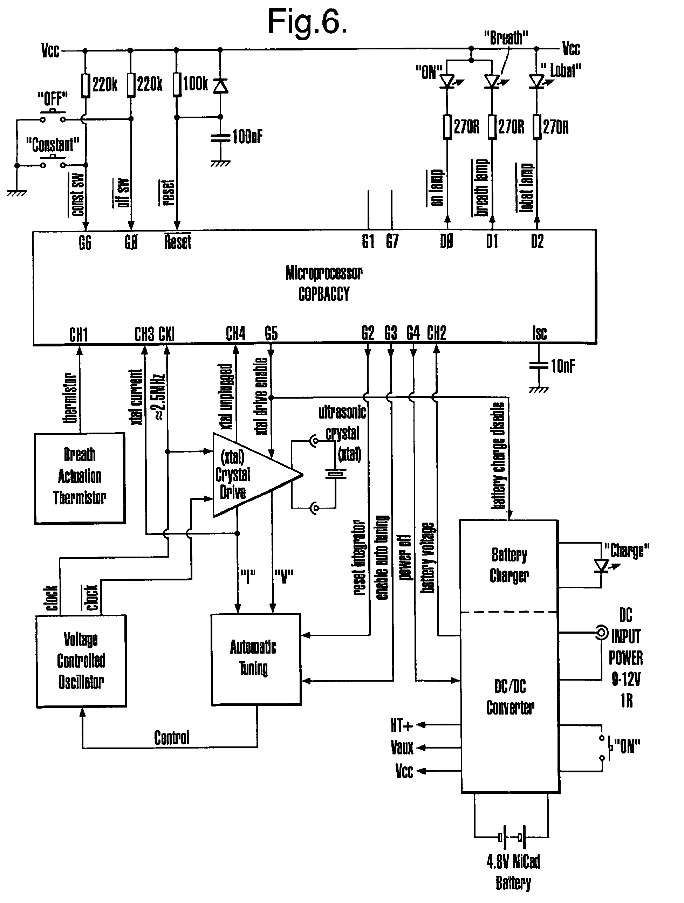

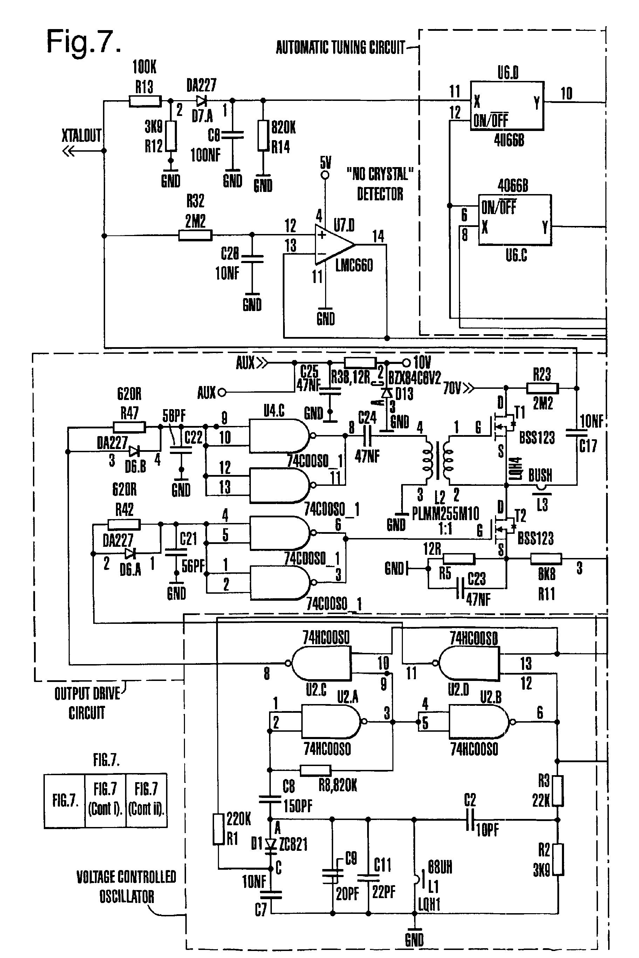

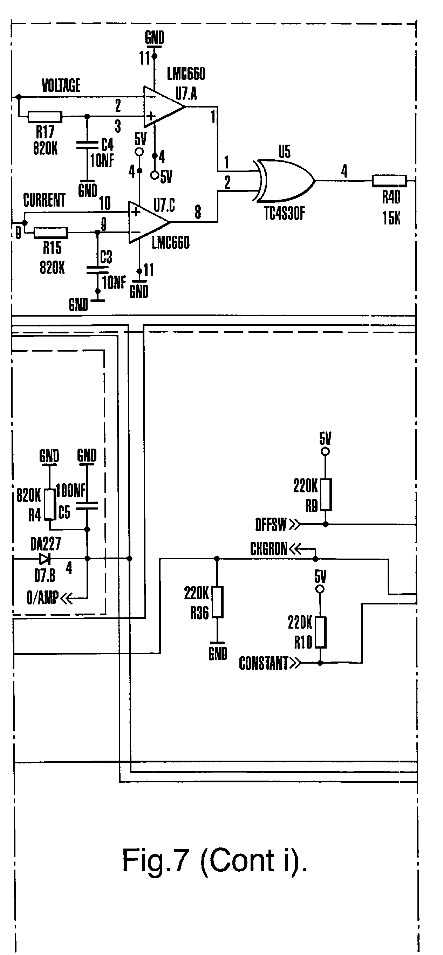

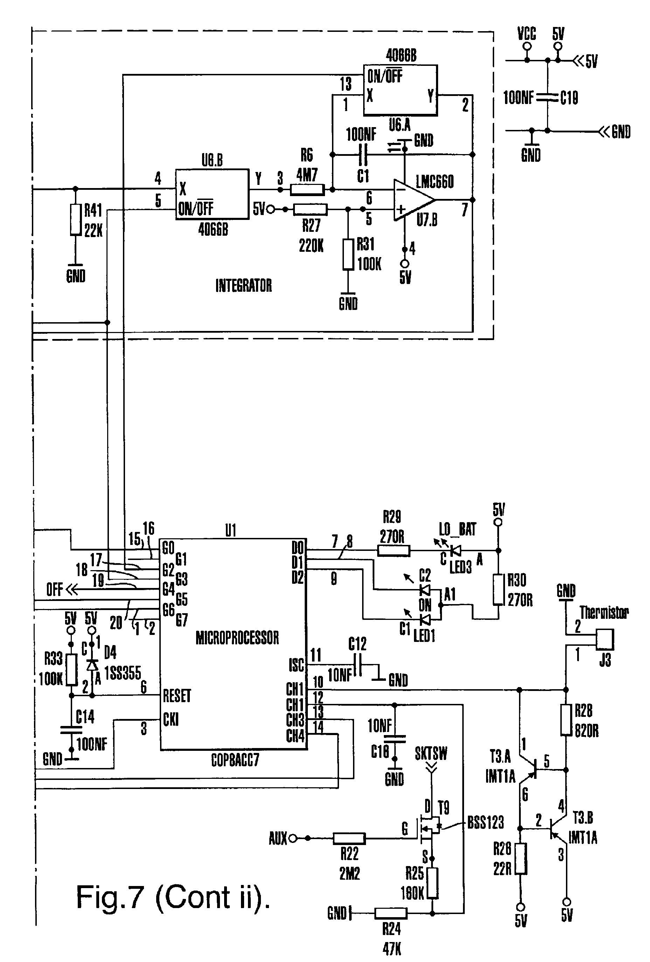

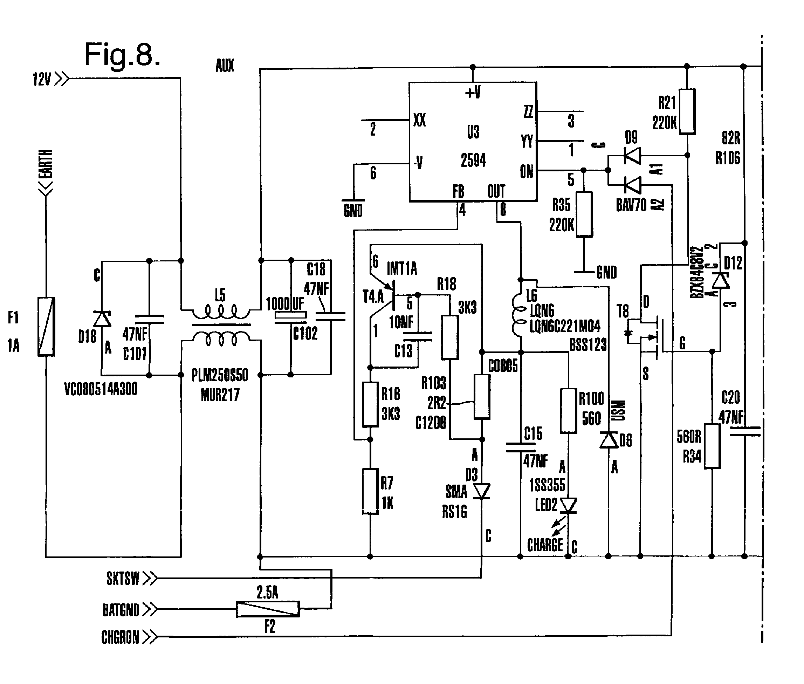

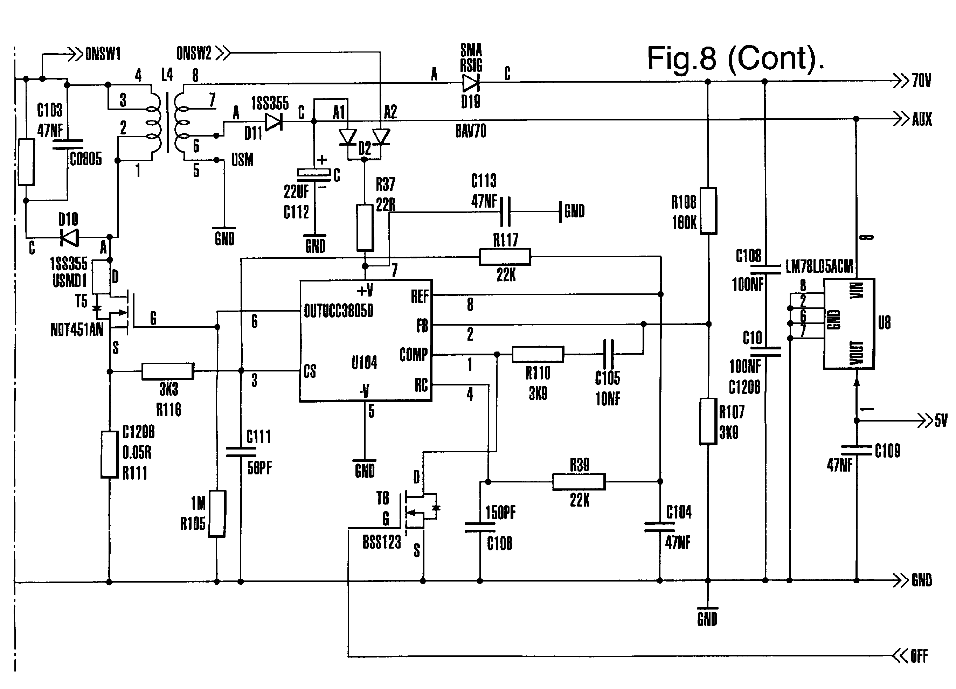

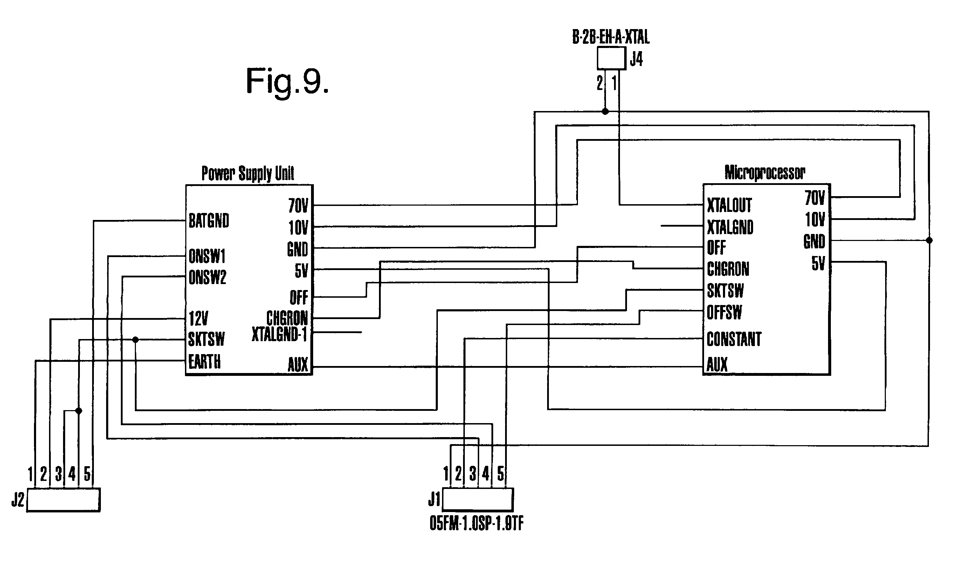

The control and power supply circuits of the present embodiment are shown in Figures 7 and 8 respectively. The relationship between these two circuits is shown in Figure 9. The general structure of the nebuliser circuitry is shown in Figure 6. An automatic tuning circuit (see Figure 7) is comprised in the control circuitry for automatically tuning transducer oscillation to a specific frequency. This tuning circuit operates on the basis of the transducer current/voltage and frequency relationship shown in Figure 5. With reference to Figure 5, it will be seen that the current passing through a transducer varies with frequency and falls to a minimum at the parallel (i.e. anti-resonance) frequency fₐ of the transducer. In addition, it will be seen that the voltage across the transducer at frequencies in the region of the parallel frequency fₐ increases with frequency. This current/voltage and frequency relationship is characteristic of ultrasonic transducers and is used in the present nebuliser to provide directional control to the automatic tuning circuit.

The aforementioned transducer characteristic may be conveniently used to automatically tune to the anti-resonance frequency. The tuning circuit comprises two comparators for determining the direction of change (i.e. increase or decrease) of either transducer voltage or current with frequency. One of the comparators is presented with a voltage representing the magnitude of the current through the transducer, whilst the other comparator is presented with a voltage representing the magnitude of the voltage across the transducer. As will be apparent to those skilled in the art, a part of the signal applied to each comparator is delayed for comparison with the instant signal. It may then be determined whether or not the applied signal is momentarily rising or falling. It will be seen that the output of the two comparators is applied to an exclusive OR gate in order to determine whether the transducer frequency is greater than or less than the frequency at which the transducer current is a minimum (i.e. the anti-resonance frequency fₐ). On the basis of this determination, the frequency may be appropriately adjusted.

The exclusive OR gate produces a clock output of varying pulse widths which is averaged with an integrator (see Figure 7) so as to produce a signal for driving a voltage controlled oscillator. This oscillator is operable at frequencies of approximately 2.5 MHz so as to ensure that, as previously discussed, the atomized liquid is generated with the desired particle size.

Two anti-phase clocks are used to operate an output drive circuit (see Figure 7). It will be seen that the output drive circuit comprises two field-effect transistors (i.e. two FETs) arranged in a totem-pole configuration so as to allow the piezo-electric transducer of the nebuliser to be referenced to ground. A level shifting network comprising a small transformer is provided in the output drive circuit for stepping up the circuit voltage to between 40V and 100V. This higher voltage is used to drive the upper FET. With appropriate signal rectification, the illustrated output drive circuit and automatic tuning circuit generate a measured rectified and smoothed transducer voltage/current and tune the oscillator to the required anti-resonance frequency.

In alternative embodiments, the circuitry of the nebuliser may be configured so as to automatically tune to a frequency which is offset from the anti-resonance frequency fₐ. This is achieved by manipulating the transducer voltage and current magnitudes so as to generate a frequency curve having a maximum or minimum occurring at a frequency other than that at which the minimum current through the transducer is found. This new maximum or minimum is then used as a new reference for the automatic tuning. For example, the tuning frequency can be offset by reference to the modulus of resistance (i.e. V/I) of the transducer. This is found by dividing the measured rectified and smoothed voltage across the transducer by the measured rectified and smoothed current through the transducer. A curve is thereby produced which has a maximum occurring at a frequency greater than the frequency at which the minimum current occurs (i.e. the anti-resonance frequency fₐ). The V/I function is derived in practice by a divider circuit. As an alternative to dividing the transducer voltage by the transducer current, these voltage and current variables may be either added (V+I), subtracted (V-I) or multiplied (VxI) so as to generate an offsetting of frequency from the anti-resonance frequency.

The present invention is not limited to the specific embodiments or methods described above. Alternative arrangements may be apparent to a reader skilled in the art.

## Claims

1. A nebuliser comprising an electrically energizable ultrasonic transducer having an oscillation frequency dependent current and voltage characteristic exhibiting the occurrence of a minimum magnitude of current through the transducer at an anti-resonance frequency of the transducer, the voltage across the transducer increasing with an increase in frequency within a range of frequencies including said anti-resonance frequency; a transducer drive system for generating a drive signal for energizing the transducer, the drive system comprising oscillation frequency control means operable to maintain the frequency of the drive signal at a specific frequency within said range of frequencies by reference to the variation of both current through the transducer and voltage across the transducer with a change of frequency within the said frequency range, the transducer being arranged to receive said drive signal and to cause physical vibrations in fluid to be nebulised when energized by said drive signal.

2. A nebuliser as claimed in claim 1, wherein the oscillation frequency control means maintains the frequency of the drive signal at a magnitude at which the variation with frequency of a function of current through the transducer is zero.

3. A nebuliser as claimed in claim 2, wherein the function of current has a zero variation with frequency at a frequency magnitude other than the anti-resonance frequency of the tranducer.

4. A nebuliser as claimed in claim 3, wherein the function of current has a zero variation with frequency at a frequency magnitude at which the yield of fluid nebulised by the transducer is substantially maximised.

5. A nebuliser as claimed in any of claims 1 to 4, wherein the oscillation frequency control means comprises a comparator for determining the direction of change with frequency of the function of current through the transducer.

6. A nebuliser as claimed in claim 5, wherein the oscillation frequency control means comprises a comparator for determining the direction of change with frequency of voltage across the transducer.

7. A nebuliser as claimed in claim 6, wherein outputs from the two comparators are applied to an exclusive OR gate for determination of the frequency magnitude at which the function of current has a zero variation with frequency.

8. A nebuliser as claimed in any of claims 2 to 7, wherein the function of current is such that the relationship of said function with frequency is identical to that of current through the transducer with frequency.

9. A nebuliser comprising means for inducing a swirling motion in nebulised fluid about a swirl axis as said nebulised fluid is extracted from the nebuliser, the angular velocity of the swirling fluid being sufficient to result in undesirably large nebulised particles moving in a direction offset to the direction of swirl and thereby away from the swirl axis.

10. A nebuliser comprising a demand prediction system which determines the period of a cyclical demand for nebulised fluid by monitoring fluid flow with detection means so as to determine the start of consecutive demand cycles and compensating for lag between the actual start of a demand cycle and the detected start of said cycle by calculating the start of a next demand cycle as being a length of time from the detected start of a current demand cycle where said length of time is equal to the determined period less a fixed length of time.
